# EUROPEAN PATENT APPLICATION

(11) **EP 4 729 920 A1**
(43) Date of publication of application: **22.04.2026**
(21) Application number: 24856064.1
(22) Date of filing: 05.03.2024
(51) Int. Cl.: G01N 11/14, E04G 21/08, G01N 11/00, G01N 33/38

(54) **FLUIDIZATION MEASURING INSTRUMENT**

(30) Priority: 18.08.2023 JP 2023133191
(71) Applicant: Japan Aviation Electronics Industry, Limited, Tokyo 150-0043 (JP)
(72) Inventor: ICHIKAWA, Shintaro, Tokyo 150-0043 (JP); TAMURA, Hironori, Tokyo 150-0043 (JP); YAMANE, Kohei, Tokyo 150-0043 (JP); YAMAZAKI, Shinji, Tokyo 103-0016 (JP); TANAKA, Masashi, Tokyo 103-0016 (JP); NOBORU, Satoshi, Tokyo 103-0016 (JP); HASHIDA, Masaya, Tokyo 103-0016 (JP)
(74) Representative: MERH-IP Matias Erny Reichl Hoffmann Patentanwälte PartG mbB
(86) International application number: PCT/JP2024/008191
(87) International publication number: WO 2025/041368

(57) **Abstract**

Fluidity of a material can be stably measured and a sensor used for the measurement can be reliably retrieved. A fluidization measuring instrument 1 includes: an insertion and extraction jig 10 which includes an end portion 11 and an end portion 12; a shaft portion 20 which includes an end portion 21 and an end portion 22 and is inserted into a material D together with the insertion and extraction jig 10 with the end portion 21 connected to the insertion and extraction jig 10; a rotating body 30 which includes an end portion 31 and an end portion 32 and is connected with the end portion 22 at a mounting position T so as to be rotatable with respect to the insertion and extraction jig 10 using the shaft portion 20 as a rotating shaft; and a sensor unit 40 which is capable of detecting rotation of the rotating body. The rotating body 30 is configured so that when the rotating body 30 is entirely inserted into the material D while maintaining a first posture, in which the end portion 32 is deeper than the end portion 31 in a depth direction, a center of buoyancy Cb, a center of gravity Cg, and the mounting position T are set so as to change a posture from the first posture to a second posture, in which the end portion 31 is deeper than the end portion 32 in the depth direction, along with the rotation associated with fluidization of the material D.

## Description

### [TECHNICAL FIELD]

The present disclosure relates to a fluidization measuring instrument for measuring a degree of fluidization of a material.

### [BACKGROUND ART]

As a prior art of a method for determining completion of concrete compaction, there is a method by which completion of concrete compaction is easily determined when a vibrator is inserted into concrete placed in a formwork to apply vibrations to the concrete to compact it, as illustrated in FIG. 1 (for example, see Patent Literature 1). In this method, floating bodies F floating up in concrete C according to the compacting degree of the concrete C by compaction of the concrete C in a formwork M by a vibrator V are set on a bottom of the portion of determining the concrete compaction completion in the formwork M before concrete placing. From this state, the concrete C is placed in the formwork M, compacted by the vibrator V, and the completion of concrete compaction is determined with floating up of the floating bodies F.

### [PRIOR ART LITERATURE]

### [PATENT LITERATURE]

Patent Literature 1: Japanese Patent Application Laid Open No. 2019-183392

### [SUMMARY OF THE INVENTION]

### [PROBLEMS TO BE SOLVED BY THE INVENTION]

The above-mentioned method for determining completion of concrete compaction in Patent Literature 1, however, has a risk that the floating bodies cannot be retrieved if the floating bodies do not float up.

In light of the above-described problem, the present disclosure aims to provide a fluidization measuring instrument which can stably measure a degree of fluidity of a material fluidized by shaking or heating, and of which a sensor used for the measurement can be more reliably retrieved.

### [MEANS TO SOLVE THE PROBLEMS]

To solve the above-described problem, a fluidization measuring instrument according to the present embodiment is a fluidization measuring instrument for measuring a degree of fluidization of a material which is fluidized by shaking or heating. The fluidization measuring instrument includes: an insertion and extraction jig which includes a first end portion and a second end portion, the second end portion being positioned opposite to the first end portion and being to be inserted into the material before performing a process of shaking or heating; a shaft portion which includes one end and the other end positioned opposite to the one end, and which is inserted into the material together with the insertion and extraction jig with the one end connected to the insertion and extraction jig; a rotating body which includes a third end portion and a fourth end portion positioned opposite to the third end portion, and which is connected with the other end of the shaft portion, at a mounting position provided between the third end portion and the fourth end portion, so as to be rotatable with respect to the insertion and extraction jig using the shaft portion as a rotating shaft; and a sensor unit which is capable of detecting rotation of the rotating body. The rotating body is configured so that when the rotating body is entirely inserted into the material while maintaining a first posture, in which the fourth end portion is deeper than the third end portion in a depth direction, a position of a center of buoyancy of the rotating body, a position of a center of gravity of the rotating body, and the mounting position are set so as to change a posture from the first posture to a second posture, in which the third end portion is deeper than the fourth end portion in the depth direction, along with the rotation associated with fluidization of the material.

### [EFFECTS OF THE INVENTION]

According to the fluidization measuring instrument of the present embodiment, a degree of fluidity of a material fluidized by shaking or heating can be stably measured and a sensor used for the measurement can be more reliably retrieved.

### [BRIEF DESCRIPTION OF THE DRAWINGS]

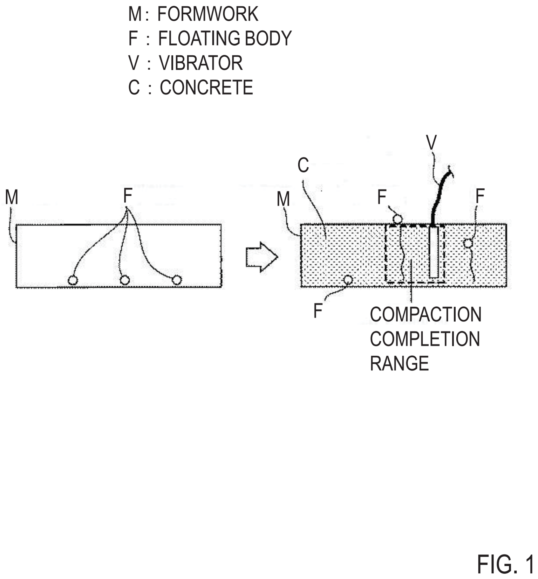

[FIG. 1] FIG. 1 is a diagram illustrating a prior art of a method for determining completion of concrete compaction.
[FIG. 2] FIG. 2 is a top view of a fluidization measuring instrument 1 according to a first embodiment installed in a formwork 200 into which fresh concrete has been poured, as viewed from above.
[FIG. 3] FIG. 3 is a diagram illustrating an A-A section in FIG. 2.
[FIG. 4] FIG. 4 is a flowchart showing a fluidization measuring method using the fluidization measuring instrument 1.
[FIG. 5] FIG. 5(a) is a perspective view of the fluidization measuring instrument 1 according to the first embodiment and FIG. 5(b) is an A-A (FIG. 2) sectional view of the fluidization measuring instrument 1.
[FIG. 6] FIG. 6 is a front elevational view of a rotating body 30 viewed from an insertion and extraction jig 10 side.
[FIG. 7] FIG. 7(a) is a diagram illustrating a volume V1 and FIG. 7(b) is a diagram illustrating a volume V2, an outer shape of which is the same shape as FIG. 7(a).
[FIG. 8] FIG. 8 illustrates cases in which a hole portion 30H of the rotating body 30 is formed closer to an end portion 32, where FIG. 8(a) illustrates an example in which a center of gravity C_{g} is closer to the hole portion 30H than a center of buoyancy C_{b} and FIG. 8(b) illustrates an example in which the center of buoyancy C_{b} is closer to the hole portion 30H than the center of gravity C_{g}.
[FIG. 9] FIG. 9 illustrates cases in which the hole portion 30H of the rotating body 30 is formed closer to an end portion 31, where FIG. 9(a) illustrates an example in which the center of buoyancy C_{b} is closer to the hole portion 30H than the center of gravity C_{g} and FIG. 9(b) illustrates an example in which the center of gravity C_{g} is closer to the hole portion 30H than the center of buoyancy C_{b}.
[FIG. 10] FIG. 10 illustrates cases in which the hole portion 30H of the rotating body 30 is formed between the center of gravity C_{g} and the center of buoyancy C_{b}, where FIG. 10(a) illustrates an example in which the center of gravity C_{g} is closer to the end portion 31 than the center of buoyancy C_{b} and FIG. 10(b) illustrates an example in which the center of buoyancy C_{b} is closer to the end portion 31 than the center of gravity C_{g}.
[FIG. 11] FIG. 11(a) is a perspective view of the fluidization measuring instrument 1 in which a magnetic system is adopted for a sensor unit 40 and FIG. 11(b) is a side view of the same.
[FIG. 12] FIG. 12 is a front elevational view illustrating an example of the rotating body 30 viewed from the insertion and extraction jig 10 side in a configuration adopting the magnetic system for the sensor unit 40.
[FIG. 13] FIG. 13 is a front elevational view illustrating an example of the insertion and extraction jig 10 viewed from the rotating body 30 side in the configuration adopting the magnetic system for the sensor unit 40.
[FIG. 14] FIG. 14 is a diagram illustrating an example of a method for connecting a shaft portion 20 and the rotating body 30 according to a modification of the first embodiment.
[FIG. 15] FIG. 15 is a diagram illustrating another example of the method for connecting the shaft portion 20 and the rotating body 30 according to the modification of the first embodiment.
[FIG. 16] FIG. 16 is a diagram illustrating an example of a method for connecting the insertion and extraction jig 10 and the shaft portion 20 according to the modification of the first embodiment.
[FIG. 17] FIG. 17 is a diagram illustrating other configurations of the insertion and extraction jig 10.
[FIG. 18] FIG. 18(a) is a perspective view of a fluidization measuring instrument 1A according to a second embodiment and FIG. 18(b) is a side view of the same.
[FIG. 19] FIG. 19 is a front elevational view of a rotating body 30A viewed from an insertion and extraction jig 10A side.
[FIG. 20] FIG. 20 is a front elevational view of the insertion and extraction jig 10A viewed from a rotating body 30A side.
[FIG. 21] FIG. 21 is a flowchart showing a fluidization measuring method using the fluidization measuring instrument 1A.
[FIG. 22] FIG. 22 is a diagram illustrating an example of a positional relationship between the center of gravity C_{g}, the center of buoyancy C_{b}, and the shaft portion 20 in the rotating body 30A.

### [DETAILED DESCRIPTION OF THE EMBODIMENTS]

A fluidization measuring method according to the present disclosure and an embodiment of a fluidization measuring instrument 1 used for the method will be described below with reference to the accompanying drawings. In the following description, components having the mutually-same functions will be provided with the mutually-same reference characters and duplicate description thereof will be omitted.

### <Fluidization measuring method>

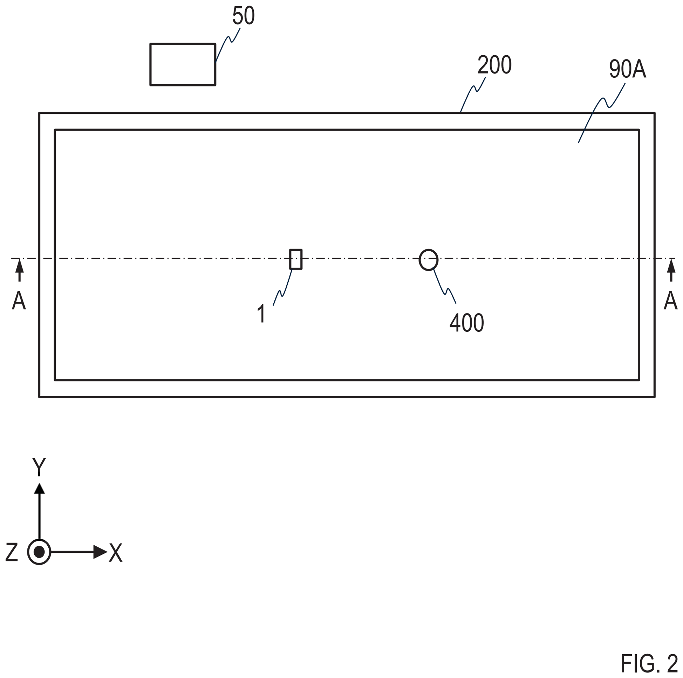

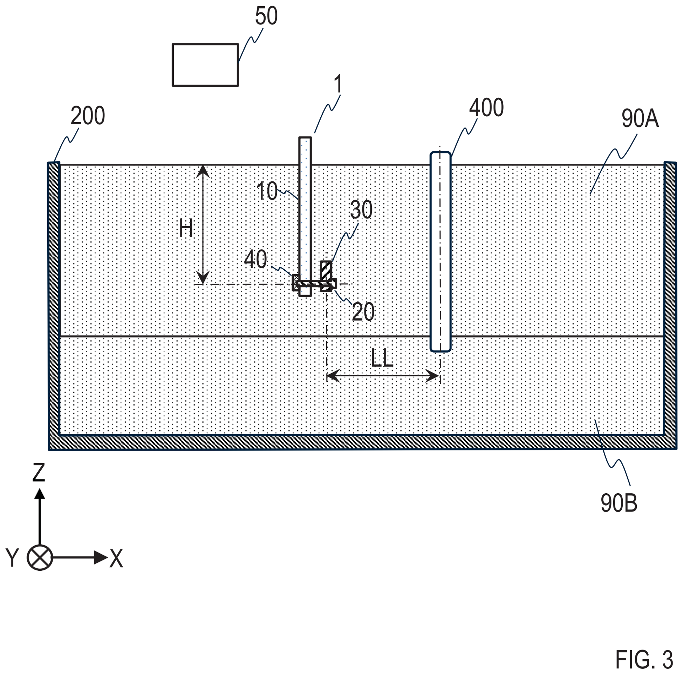

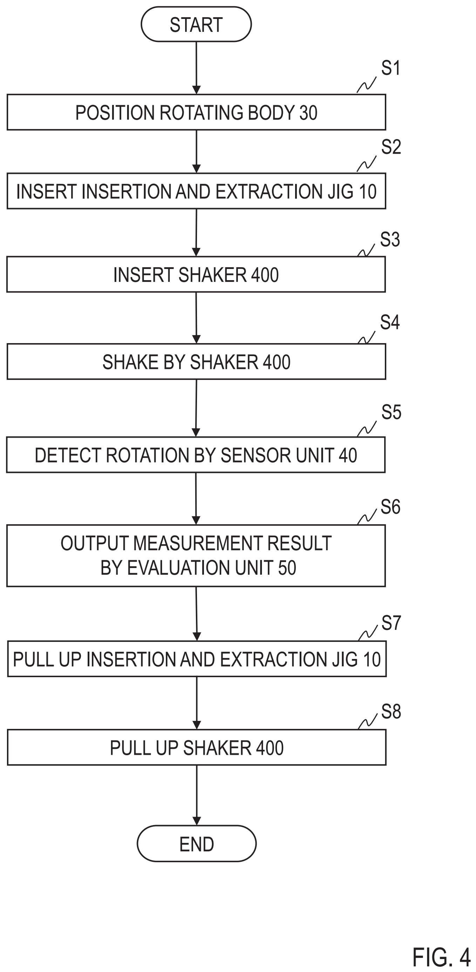

FIG. 2 is a top view of the fluidization measuring instrument 1 according to a first embodiment installed in a formwork 200 into which fresh concrete has been poured, as viewed from above. FIG. 3 is a diagram illustrating an A-A section in FIG. 2. FIG. 4 is a flowchart showing the fluidization measuring method using the fluidization measuring instrument 1.

In the fluidization measuring method according to the present disclosure, for example, a degree of fluidization of fresh concrete which has been poured into the formwork 200 is measured. Specifically, the fluidization measuring instrument 1 is inserted into the fresh concrete and the fresh concrete is shaken by a shaker 400. In a state in which the fresh concrete is fluidized by this shaking, a rotating body 30 rotates about a shaft portion 20 as a rotating shaft depending on a degree of fluidization. The degree of fluidization is measured by measuring the rotation of the rotating body 30 with a sensor unit 40. When a measurement target is a material fluidized by heating, a heater is used instead of the shaker 400. The following description will be given taking fresh concrete as an example of the measurement target.

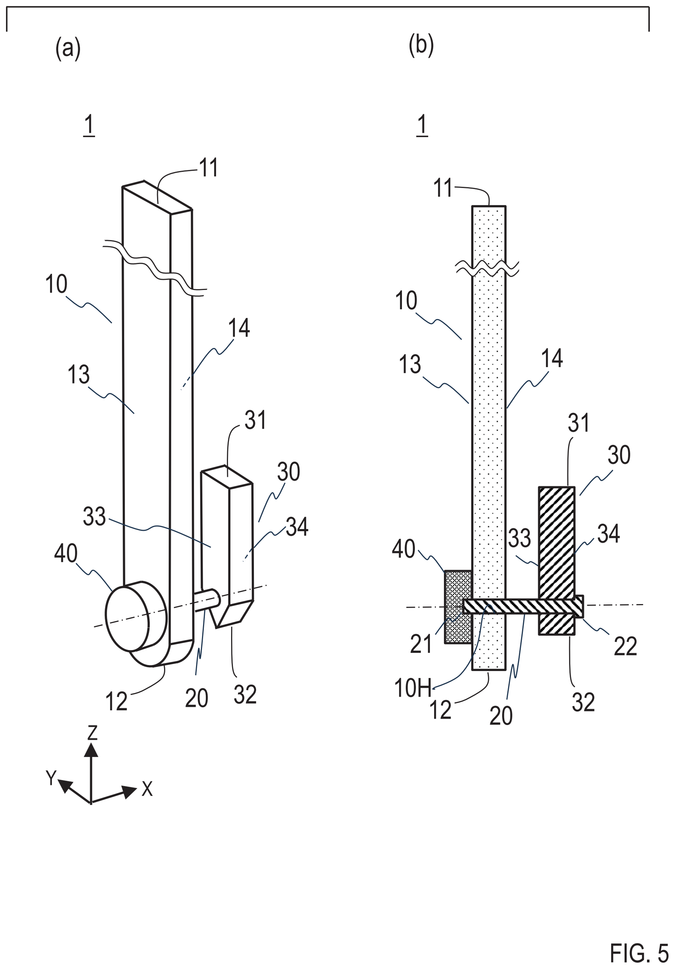

As illustrated in FIG. 4, in the fluidization measuring method of the present disclosure, positioning is first performed to obtain a posture of the rotating body 30 in which an end portion 31 is positioned in an upward direction (Z direction in FIG. 5) and an end portion 32 is positioned in a downward direction (-Z direction in FIG. 5) with respect to a posture of the fluidization measuring instrument 1 in which an end portion 11 is positioned in the upward direction (Z direction in FIG. 5) and an end portion 12 is positioned in the downward direction (-Z direction in FIG. 5) (hereinafter, also referred to as the "first posture") (step S1). The end portion 11 and the end portion 12 will be described later.

Next, while maintaining the first posture, the end portion 12 side of the insertion and extraction jig 10 is inserted into an upper layer 90A of the fresh concrete (step S2). Accordingly, in addition to a portion of the insertion and extraction jig 10, the shaft portion 20, the rotating body 30, and the sensor unit 40 are inserted into the upper layer 90A of the fresh concrete, as illustrated in FIG. 3. Through step S2, the rotating body 30 can be reliably placed at a desired measurement position. In the unlikely event that step S2 causes the rotating body 30 to rotate and no longer maintain the first posture, another jig is used to position the rotating body 30 so that the rotating body 30 is in the first posture.

Then, as illustrated in FIG. 3, the shaker 400 during or before shaking is inserted at a position separated by a length LL from the rotating body 30 (step S3). The length LL and a depth H described later are lengths determined to be preferable for measuring a degree of fluidization of a preadjusted measurement target. The length LL is preferably within a range in which the rotating body 30 is not easily affected by the shaking of the shaker 400 itself and the shaking required for fluidization is transmitted.

When the measurement target is fresh concrete, the insertion depth of the rotating body 30 is set to a position of the depth H from an upper surface of the upper layer 90A, which has been poured above (Z direction side) a lower layer 90B of the fresh concrete which has already been poured the first time and compacted, as illustrated in FIG. 3. Here, the fluidization measuring instrument 1 can also be used for the fluidization measuring method for fresh concrete poured for the first time or fresh concrete poured for the third time or later. Through step S3, the rotating body 30 is positioned away from the shaker 400 by LL and at the depth H.

The shaker 400 of the present embodiment is composed of a general shaker for compacting fresh concrete. When fresh concrete has two layers, the shaker 400 penetrates through the upper layer 90A which is to be next compacted, and a portion near its tip, for example, approximately 100 mm, is inserted into the lower layer 90B, which has already been compacted. When fresh concrete which is an evaluation target is the fresh concrete poured for the first time, the shaker 400 is required not to come into contact with the bottom. Though depending on a diameter and shaking output strength of the shaker 400, when the diameter of the shaker 400 is 50 mm, a range of, for example, a radius of approximately 250 mm centered on the shaker 400 is a range where shaking can be transmitted to compact the fresh concrete.

Next, the fresh concrete is shaken by the shaker 400 (step S4). The shaking of the shaker 400 causes the fresh concrete to start fluidization. The fresh concrete is compacted by the shaking of the fresh concrete with the shaker 400. When the shaker 400 operates in the fresh concrete, the shaking causes particles constituting the fresh concrete to fall into gaps between the particles. The falling of the particles into the gaps increases water pressure in the gaps. As the water pressure increases, a frictional force between the particles decreases, and buoyancy becomes more dominant than the frictional force in the upper layer 90A of the fresh concrete. The fresh concrete is thus fluidized.

As the fluidization of the upper layer 90A progresses, the frictional force around the rotating body 30 decreases. In step S4, based on buoyancy or gravity acting on the rotating body 30, the rotating body 30 in the first posture (the end portion 31 is in the upward direction (Z direction in FIG. 5) and the end portion 32 is in the downward direction (-Z direction)) rotates about the shaft portion 20 as a rotating shaft so that the end portion 32 moves toward the upward direction (Z direction). The rotating body 30 thus tries to change from the first posture to a second posture (the end portion 31 is in the downward direction (-Z direction) and the end portion 32 is in the upward direction (Z direction)).

While the rotating body 30 moves from the first posture to the second posture or on the way from the first posture to the second posture, the sensor unit 40 detects rotation of the rotating body 30 (step S5). That is, the sensor unit 40 detects the degree of the rotation of the rotating body 30 which rotates about the shaft portion 20 as a rotating shaft. A detection result of the sensor unit 40 is sent to an evaluation unit 50 connected by wire or wirelessly.

Subsequently, the evaluation unit 50 outputs a measurement result, which allows evaluation of the degree of fluidization of the measurement target, based on the detection result received from the sensor unit 40 (step S6). For example, the degree of the rotation of the rotating body 30 may be displayed in chronological order, or a graph of a relationship between a predetermined degree of rotation and a degree of fluidization of a measurement target may be displayed. However, these are merely examples, and any output may be used as long as the output is a measurement result which allows evaluation of the degree of fluidization of the measurement target. A user evaluates the degree of fluidization of the measurement target through step S6.

When the user determines that a desired degree of fluidization is achieved based on the output of the measurement result of step S6, the insertion and extraction jig 10 is pulled up (step S7). The insertion and extraction jig 10 is connected with the shaft portion 20 and the shaft portion 20 is connected with the rotating body 30 as described later. Accordingly, the rotating body 30 can be reliably retrieved by pulling up the insertion and extraction jig 10 by step S7.

When the measurement target is fresh concrete, step S7 is performed before the time when compaction is predicted to be completed. Specifically, it is performed several seconds to several tens of seconds before, preferably 1 to 10 seconds before, and more preferably 1 to 5 seconds before the time when the compaction is predicted to be completed. On the other hand, the operation of the shaker 400 in step S4 is continuously performed until the time when the compaction is predicted to be completed and subsequently, the shaker 400 is pulled up (step S8).

For example, if a graph of an acceleration of the rotating body 30 is obtained in step S6, shaking time when the compaction is predicted to be completed is set to be up to time where an extension line of a gradient set from 1 to a dozen seconds, which can be read from the graph, reaches a preset composite acceleration at which the compaction is predicted to be completed. The composite acceleration at which the compaction is predicted to be completed is set based on a relationship between the composite acceleration and quality of the concrete after curing. The fluidization measuring method is thus ended.

The embodiment of the fluidization measuring instrument 1 used for the above-described fluidization measuring method will be described below.

### <First embodiment>

FIG. 5(a) is a perspective view of the fluidization measuring instrument 1 according to the first embodiment and FIG. 5(b) is an A-A (FIG. 2) sectional view of the fluidization measuring instrument 1. The fluidization measuring instrument 1 according to the first embodiment of the present disclosure measures a degree of fluidization of a material (material D) which is fluidized by shaking or heating. The fluidization measuring instrument 1 includes, for example, the insertion and extraction jig 10, the shaft portion 20, the rotating body 30, and the sensor unit 40, as illustrated in FIGs. 5(a) and 5(b).

### (Insertion and extraction jig 10)

The insertion and extraction jig 10 includes the end portion 11 and the end portion 12 which is positioned opposite to the end portion 11 and is to be inserted into the material D before performing a process of shaking or heating. The insertion and extraction jig 10 has, for example, an elongated rod shape having a substantially rectangular cross section. The insertion and extraction jig 10 is made of, for example, stainless steel. In the example of FIG. 5, the end portion 11 has a flat surface and the end portion 12 has a curved surface. The end portion 12 preferably has a tapered shape, such as having a pointed tip. The tapered shape may also be other tapered shapes such as a pyramidal shape or a conical shape. The end portion 12 side of the insertion and extraction jig 10 corresponds to a side inserted into the material D which is a measurement target. The end portion 11 side of the insertion and extraction jig 10 corresponds to a handle side, where a user manipulates the insertion and extraction jig 10 in order to insert the rotating body 30 into the material D and place it at a desired measurement position.

### (Shaft portion 20)

The shaft portion 20 has, for example, an elongated rod shape having a substantially circular cross section, and includes an end portion 21 and an end portion 22 which is positioned opposite to the end portion 21. The shaft portion 20 is made of, for example, SUS. The end portion 21 side is connected to the insertion and extraction jig 10 in a manner to be rotatable about the shaft portion 20 as a rotating shaft, and the end portion 22 side is fixedly connected to the rotating body 30. The shaft portion 20 is inserted into the material D together with the end portion 12 side of the insertion and extraction jig 10 so as to measure the degree of fluidization of the material D.

### (Rotating body 30)

The rotating body 30 has, for example, a substantially rectangular parallelepiped shape with a substantially rectangular cross section, and includes the end portion 31 and the end portion 32 which is positioned opposite to the end portion 31. In this example, the end portion 32 side of the rotating body 30 has a tapered shape formed as a triangular prism shape so that the rotating body 30 can be easily inserted into the material D. The tapered shape may also be other tapered shapes such as other pyramidal shapes or conical shapes. The rotating body 30 is made of, for example, SUS. The rotating body 30 is fixedly connected with the end portion 22 side of the shaft portion 20 at a predetermined position (mounting position T) provided between the end portion 31 and the end portion 32. Accordingly, the rotating body 30 is fixedly connected with the shaft portion 20 so as to be rotatable with respect to the insertion and extraction jig 10 using the shaft portion 20 as a rotating shaft.

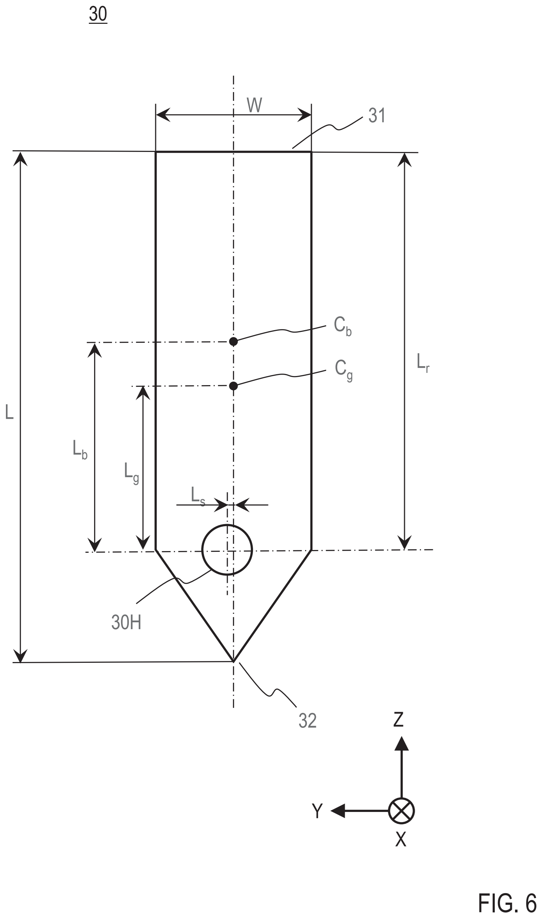

FIG. 6 is a front elevational view of the rotating body 30 viewed from an insertion and extraction jig 10 side. In this example, the length of the rotating body 30 is L, and a hole portion 30H is formed so that the shaft portion 20 is inserted at a position of a length Lᵣ from the end portion 31. The hole portion 30H corresponds to the mounting position T. In this example, a central axis of the hole portion 30H is deviated (shifted) to the left (Y direction) in FIG. 6 by a length Lₛ, from a central axis in the width direction of the rotating body 30, in order to promote the rotation direction of the rotating body 30 in one direction. In other words, the center of the mounting position T is separated from a straight line passing through the center of buoyancy of the rotating body 30 (center of buoyancy C_{b}) and the center of gravity of the rotating body 30 (center of gravity C_{g}). However, this deviation (separation) may be provided as necessary and is not essential. In this example, the center of buoyancy C_{b} of the rotating body 30 is assumed to be at a point in length L_{b} on the end portion 31 side from the central axis of the hole portion 30H. In this example, the center of gravity C_{g} of the rotating body 30 is assumed to be at a point in length L_{g} on the end portion 31 side from the central axis of the hole portion 30H. The center of buoyancy C_{b} and the center of gravity C_{g} may be deviated from the central axis in a width direction W of the rotating body 30 when a shift length Lₛ is provided, but the deviation is omitted here.

### (Sensor unit 40)

The sensor unit 40 detects the rotation of the rotating body 30. The sensor unit 40 is, for example, a rotary encoder or a resolver. When the sensor unit 40 is a rotary encoder or a resolver, the sensor unit 40 is attached on, for example, a surface 13 side of the insertion and extraction jig 10. The end portion 21 of the shaft portion 20 is inserted into the sensor unit 40 through a hole portion 10H which is formed in the insertion and extraction jig 10 and penetrates through the surface 13 and a surface 14, as illustrated in FIG. 5(b). The sensor unit 40 detects the rotation of the shaft of the shaft portion 20. The shaft portion 20 and the rotating body 30 are fixedly connected to each other and accordingly, the sensor unit 40 indirectly detects the rotation of the rotating body 30 through the detection of the rotation of the shaft portion 20 by the sensor unit 40.

### (Relative positional relationship between center of gravity C_{g}, center of buoyancy C_{b}, and mounting position T in rotating body 30)

The fluidization measuring method using the fluidization measuring instrument 1 is achieved by measuring change of the rotation of the rotating body 30 inserted into the material D. A description will be provided hereinafter on how the relative positional relationship between the center of gravity C_{g}, the center of buoyancy C_{b}, and the mounting position T, which is the mounting position of the shaft portion 20, of the rotating body 30 affects a rotational behavior of the rotating body 30 when the rotating body 30 is entirely inserted into the material D.

The rotating body 30 is inserted into the material D in the first posture in steps S1 and S2 in FIG. 4. Therefore, this first posture will be described as a basic state.

In order to facilitate understanding, the following description is divided into three cases: (1) case where the hole portion 30H (mounting position T) is formed closer to the end portion 32 than the center of buoyancy C_{b} and the center of gravity C_{g}, (2) case where the hole portion 30H is formed closer to the end portion 31 than the center of buoyancy C_{b} and the center of gravity C_{g}, and (3) case where the hole portion 30H is formed between the center of buoyancy C_{b} and the center of gravity C_{g}.

### [(1) Case where the hole portion 30H is formed closer to the end portion 32 than the center of buoyancy C_{b} and the center of gravity C_{g}]

When the rotating body 30 does not have any space thereinside and is made of a single material, the center of buoyancy C_{b} and the center of gravity C_{g} of the rotating body 30 are at the same position. When the entire inside of the rotating body 30 is not filled with the same material such as when the rotating body 30 is made of a plurality of materials with different specific gravities or when the rotating body 30 is made of a single material but has a space thereinside, the center of buoyancy C_{b} and the center of gravity C_{g} of the rotating body 30 are at different positions. This is a general phenomenon which is not limited to the rotating body 30 but is similar for other components.

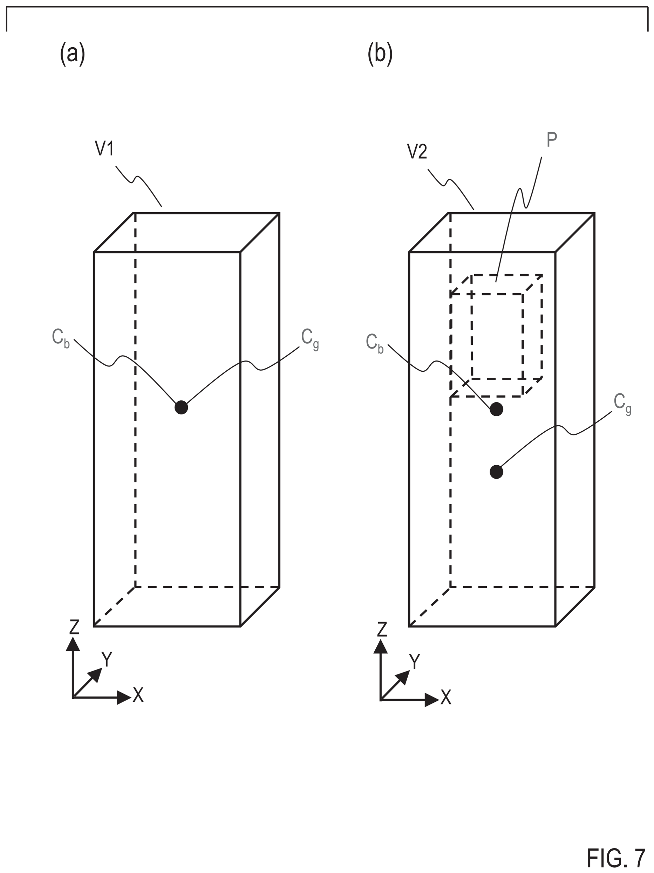

FIG. 7(a) is a diagram illustrating a volume V1 and FIG. 7(b) is a diagram illustrating a volume V2, an outer shape of which is the same shape as FIG. 7(a). The drawing shown in FIG. 7(a) is a rectangular parallelepiped with a volume of V1, and the inside is filled with a single material, such as SUS304. In this case, the positions of the center of gravity C_{g} and the center of buoyancy C_{b} coincide with each other. The drawing shown in FIG. 7(b) has an identical external shape as FIG. 7(a), and is identical in that it is filled with a single material, such as SUS304. However, it is assumed to have a space with a volume P as an internal space on the upper side (Z side) from the center, consequently having a volume of V2 (= V1-P). In this case, the center of buoyancy C_{b} in FIG. 7(b) is at the same position as in FIG. 7(a), but the center of gravity C_{g} in FIG. 7(b) moves downward (-Z side) compared to FIG. 7(a). In this way, even if the shapes are the same externally, the position of the center of gravity C_{g} can be adjusted by providing portions with different specific gravities, such as material compositions or by providing a space inside. Thus, the positions of the center of gravity C_{g}, the center of buoyancy C_{b}, and the mounting position T can be adjusted by adjusting the shape or the specific gravity of the rotating body 30.

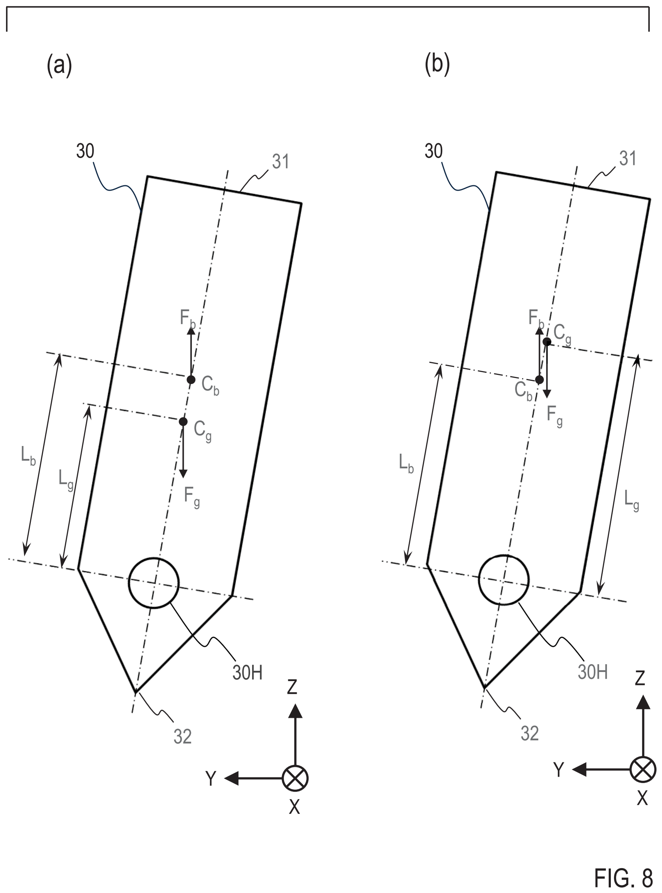

FIG. 8 illustrates cases in which the hole portion 30H of the rotating body 30 is formed closer to the end portion 32, where FIG. 8(a) illustrates an example in which the center of gravity C_{g} is closer to the hole portion 30H than the center of buoyancy C_{b} and FIG. 8(b) illustrates an example in which the center of buoyancy C_{b} is closer to the hole portion 30H than the center of gravity C_{g}. FIGs. 8(a) and 8(b) illustrate a state in which the rotating body 30 is rotated 10 degrees clockwise about the central axis of the hole portion 30H to facilitate understanding. The shift length Lₛ is not provided. In FIGs. 8(a) and 8(b), a buoyancy F_{b} acts in the upward direction (Z direction) from the center of buoyancy C_{b} and a gravity F_{g} acts in the downward direction (-Z direction) from the center of gravity C_{g}. Therefore, in order to rotate the rotating body 30 about the central axis of the hole portion 30H along with fluidization of the material D, a force is required to rotate the rotating body 30 further clockwise from the state of FIGs. 8(a) and 8(b). Here, assuming that the forces involved in the rotation of the rotating body 30 are only the gravity F_{g} and the buoyancy F_{b}, a condition F_{g}×L_{g} > F_{b}×L_{b} is required in FIGs. 8(a) and 8(b). Here, the specific gravity of the material D is denoted by S₀, and the specific gravity of the rotating body 30 is denoted by S₁. The rotating body 30 is assumed to have no space in the inside thereof. An acceleration of gravity is denoted by g. F_{b} = S₀×(volume of rotating body 30)×g is established, and F_{g} = S₁×(volume of rotating body 30)×g is established. Therefore, a condition L_{g} > (S₀/S₁)L_{b} (hereinafter also referred to as a "first relationship") is required. That is, in the above-described case of (1), a ratio between the length from the center of buoyancy C_{b} to the mounting position T and the length from the center of gravity C_{g} to the mounting position T satisfies the predetermined first relationship.

For example, if the material D is fresh concrete (specific gravity S₀ = 2.3) and the material of the rotating body 30 is SUS304 (specific gravity S₁ = 7.9), the rotating body 30 needs to be configured to satisfy L_{g} > 0.291L_{b}. For example, if the material of the rotating body 30 is polypropylene (specific gravity S₁ = 0.9), the rotating body 30 needs to be configured to satisfy L_{g} > 2.55L_{b}.

### [(2) Case where the hole portion 30H is formed closer to the end portion 31 than the center of buoyancy C_{b} and the center of gravity C_{g}]

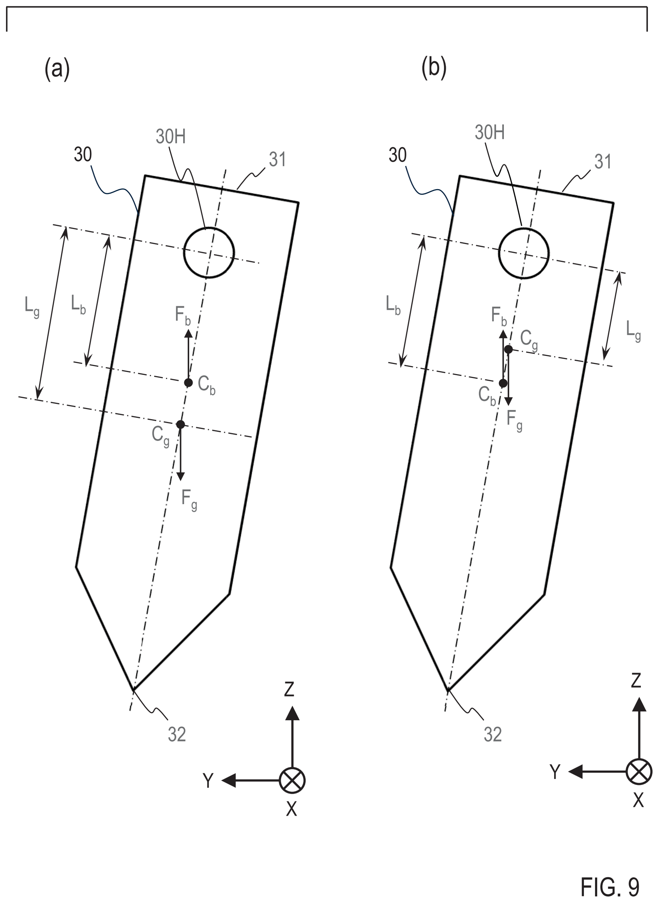

FIG. 9 illustrates cases in which the hole portion 30H of the rotating body 30 is formed closer to the end portion 31, where FIG. 9(a) illustrates an example in which the center of buoyancy C_{b} is closer to the hole portion 30H than the center of gravity C_{g} and FIG. 9(b) illustrates an example in which the center of gravity C_{g} is closer to the hole portion 30H than the center of buoyancy C_{b}. FIGs. 9(a) and 9(b) illustrate a state in which the rotating body 30 is rotated 10 degrees clockwise about the central axis of the hole portion 30H and the shift length Lₛ is not provided. In order to rotate the rotating body 30 along with fluidization of the material D, a force is required to rotate the rotating body 30 further clockwise from the state of FIG. 9. Considering a relationship of rotational moment in the same manner as in FIG. 8, a condition L_{g} < (S₀/S₁)L_{b} (hereinafter also referred to as a "second condition") is required in FIGs. 9(a) and 9(b). That is, in the above-described case of (2), a ratio between the length from the center of buoyancy C_{b} to the mounting position T and the length from the center of gravity C_{g} to the mounting position T needs to satisfy a predetermined second relationship.

For example, if the material D is fresh concrete (specific gravity S₀ = 2.3) and the material of the rotating body 30 is SUS304 (specific gravity S₁ = 7.9), the rotating body 30 needs to be configured to satisfy L_{g} < 0.291L_{b}. For example, if the material of the rotating body 30 is polypropylene (specific gravity S₁ = 0.9), the rotating body 30 needs to be configured to satisfy L_{g} < 2.55L_{b}.

### [(3) Case where the hole portion 30H is formed between the center of buoyancy C_{b} and the center of gravity C_{g}]

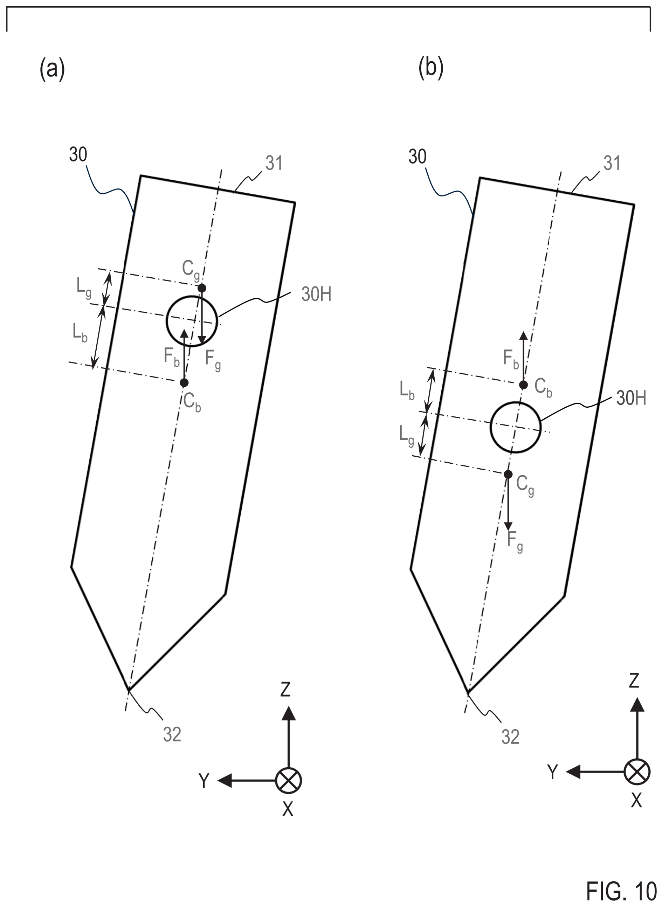

FIG. 10 illustrates cases in which the hole portion 30H of the rotating body 30 is formed between the center of gravity C_{g} and the center of buoyancy C_{b}, where FIG. 10(a) illustrates an example in which the center of gravity C_{g} is closer to the end portion 31 than the center of buoyancy C_{b} and FIG. 10(b) illustrates an example in which the center of buoyancy C_{b} is closer to the end portion 31 than the center of gravity C_{g}. FIGs. 10(a) and 10(b) illustrate a state in which the rotating body 30 is rotated 10 degrees clockwise about the central axis of the hole portion 30H and the shift length Lₛ is not provided. In order to rotate the rotating body 30 along with fluidization of the material D, a force is required to rotate the rotating body 30 further clockwise from the state of FIG. 10. Considering the relationship of rotational moment in the same manner as in FIG. 8, both the buoyancy F_{b} and the gravity F_{g} act as forces which assist the rotating body 30 to rotate further clockwise about the shaft portion 20 as the central axis, in FIG. 10(a). Both the buoyancy F_{b} and the gravity F_{g} act as forces which assist the rotating body 30 to rotate counterclockwise about the shaft portion 20 as the central axis, in FIG. 10(b). That is, FIG. 10(a) is considered to be a favorable positional relationship for detecting the state of rotation of the rotating body 30. On the other hand, FIG. 10(b) is considered to be an unsuitable positional relationship for detecting the state of rotation of the rotating body 30. That is, in the above-described case of (3), the rotating body 30, in the first posture, needs to satisfy a relationship in which the center of buoyancy Cb is positioned closer to the end portion 32 than the mounting position T and the center of gravity Cg is positioned closer to the end portion 31 than the mounting position T (hereinafter also referred to as a "third relationship").

To summarize the above, (1) when the position of the hole portion 30H, which is the mounting position of the shaft portion 20, in the rotating body 30 is closer to the end portion 32 than the center of buoyancy C_{b} and the center of gravity C_{g}, it is necessary to satisfy the first relationship in which L_{g} > (S₀/S₁)L_{b}. (2) When the position of the hole portion 30H in the rotating body 30 is closer to the end portion 31 than the center of buoyancy C_{b} and the center of gravity C_{g}, it is necessary to satisfy the second relationship in which L_{g} < (S₀/S₁)L_{b}. (3) When the position of the hole portion 30H in the rotating body 30 is between the center of buoyancy C_{b} and the center of gravity C_{g}, it is necessary to satisfy the third relationship in which the center of buoyancy C_{b} is closer to the end portion 32 and the center of gravity C_{g} is closer to the end portion 31.

However, the actual phenomenon is affected by other factors, such as frictional resistance between the insertion and extraction jig 10 and the shaft portion 20. In particular, in fresh concrete, buoyancy becomes more dominant than a frictional force in the upper layer 90A of the fresh concrete along with fluidization, but there are still situations in which the buoyancy is not fully exerted due to the frictional force. Therefore, it is preferable to utilize the above-mentioned (1) to (3) as basic standards, and in practice, to determine the relative positional relationship between the center of gravity C_{g}, the center of buoyancy C_{b}, and the mounting position T through repeated experiments and so forth. That is, the rotating body 30 of the present embodiment needs to be configured so that when the rotating body 30 is entirely inserted into the material D while maintaining the first posture in which the end portion 32 is deeper than the end portion 31 in the depth direction, the position of the center of buoyancy Cb of the rotating body 30, the position of the center of gravity Cg of the rotating body 30, and the mounting position T are set so as to change the posture from the first posture to the second posture in which the end portion 31 is deeper than the end portion 32 in the depth direction along with the rotation associated with fluidization of the material D. In the determined positional relationship, a relationship between the change of the rotation of the rotating body 30 and a degree of fluidization of the material D is to be acquired in advance.

The embodiment of the fluidization measuring instrument according to the present disclosure has been described above. By configuring the fluidization measuring instrument 1 as described above, the rotating body 30 can be placed at a desired measurement position in relation to the shaker 400 by manipulating the insertion and extraction jig 10. The rotating body 30 can be rotated about the shaft portion 20 as the central axis with respect to the insertion and extraction jig 10, being able to measure the degree of fluidization of the material D. After the measurement, the rotating body 30 can be reliably retrieved by pulling up the insertion and extraction jig 10. Namely, according to the fluidization measuring instrument of the present embodiment, a degree of fluidity of a material fluidized by shaking or heating can be stably measured and a sensor used for the measurement can be more reliably retrieved.

### <Modification of first embodiment>

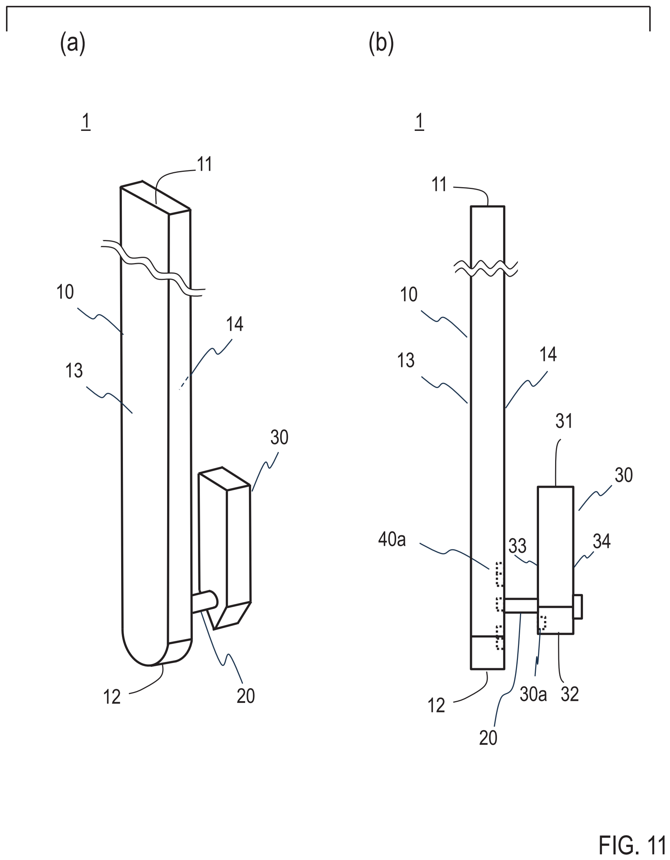

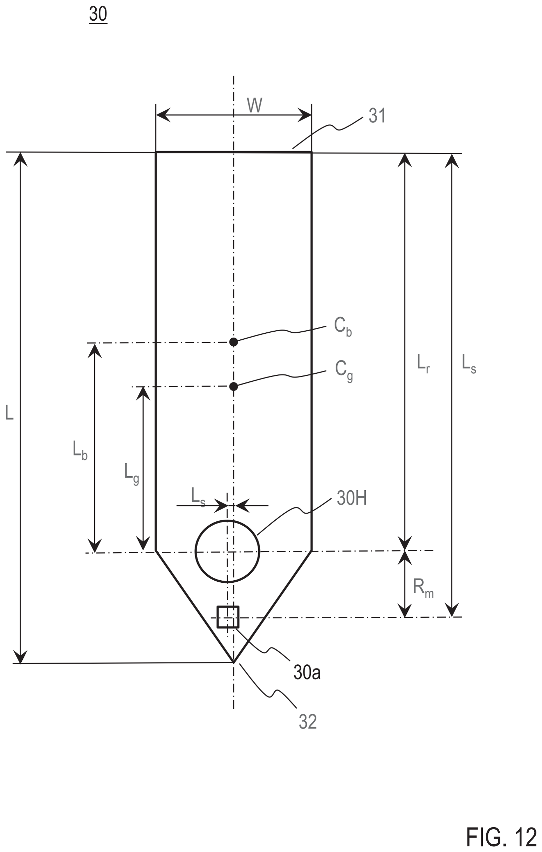

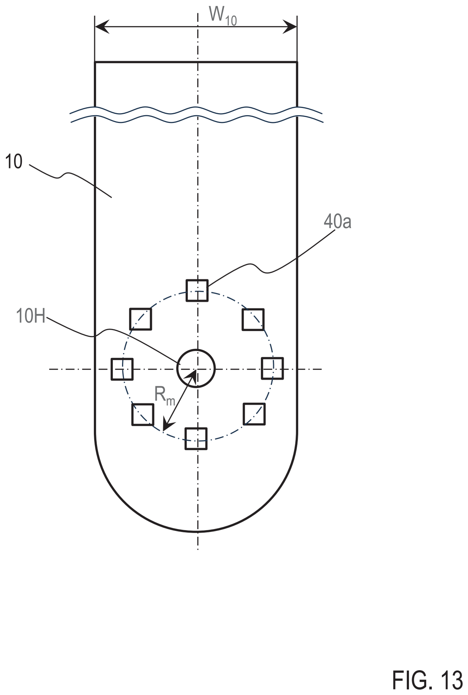

The fluidization measuring instrument 1 may adopt a magnetic system for the sensor unit 40. FIG. 11(a) is a perspective view of the fluidization measuring instrument 1 in which a magnetic system is adopted for the sensor unit 40 and FIG. 11(b) is a side view of the same. In FIG. 11(b), magnetic sensors 40a and a magnet 30a, which are not actually visible in a side view, are indicated by dotted lines. FIG. 12 is a front elevational view illustrating an example of the rotating body 30 viewed from the insertion and extraction jig 10 side in a configuration adopting the magnetic system for the sensor unit 40. FIG. 13 is a front elevational view illustrating an example of the insertion and extraction jig 10 viewed from the rotating body 30 side in the configuration adopting the magnetic system for the sensor unit 40. In FIGs. 11 and 12, as an example, the mounting position T is provided closer to the end portion 32 of the rotating body 30, under the above-mentioned condition of (1). However, the present modification may employ any of the above-mentioned conditions of (1) to (3).

As illustrated in FIGs. 11(b) and 12, the magnet 30a is embedded in the end portion 32 side of a surface 33 of the rotating body 30 of the present modification. As illustrated in FIGs. 11(b) and 13, the plurality of magnetic sensors 40a for detecting magnetism of the magnet 30a are embedded around the hole portion 10H, which is a connection portion with the shaft portion 20 on the surface 14 side, in the insertion and extraction jig 10. According to the configuration of the present modification, as the rotating body 30 rotates about the shaft portion 20 as a central axis, the magnet 30a embedded in the rotating body 30 also rotates about the shaft portion 20 as the central axis. The rotational movement of the magnet 30a is detected by the magnetic sensors 40a embedded in the insertion and extraction jig 10.

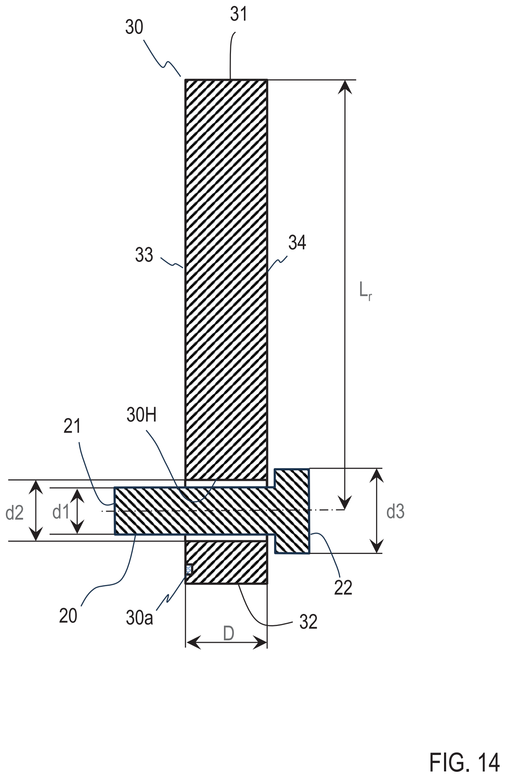

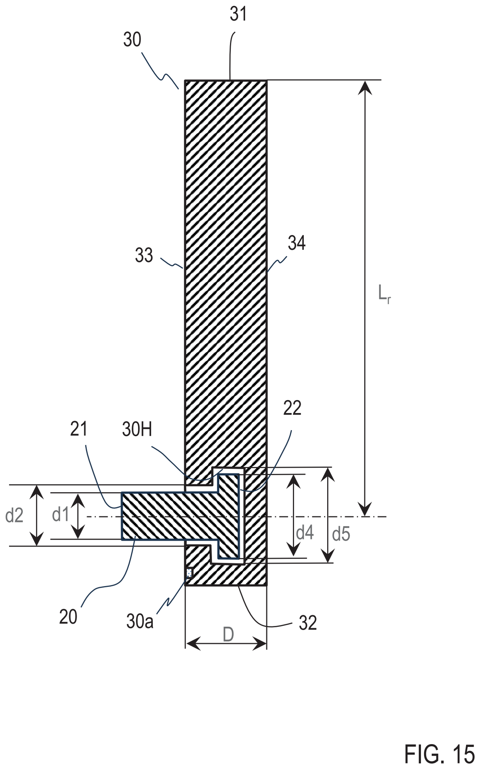

FIG. 14 is a diagram illustrating an example of a method for connecting the shaft portion 20 and the rotating body 30 according to the modification of the first embodiment. FIG. 15 is a diagram illustrating another example of the method for connecting the shaft portion 20 and the rotating body 30 according to the modification of the first embodiment. FIGs. 14 and 15 illustrate the shaft portion 20 and the rotating body 30 on the A-A (FIG. 2) sectional view, as in FIG. 5(b). When the magnetic sensor 40a is used as the sensor unit 40, as an example, it is conceivable that the rotating body 30 is connected to the shaft portion 20 in a rotatable manner. In this configuration, as illustrated in FIG. 14, the hole portion 30H of the rotating body 30 may be formed as a through hole having a diameter d2, which is larger than a diameter d1 of the shaft portion 20, so as to insert the shaft portion 20 therethrough. In this case, in order to make the connection on the end portion 22 side more secure, it is preferable to configure the end portion 22 to have a diameter d3 which is larger than the diameter d2. Here, when the rotating body 30 is connected to the shaft portion 20 in a rotatable manner, the shaft portion 20 and the insertion and extraction jig 10 are fixedly connected to each other.

As another example of connecting the rotating body 30 to the shaft portion 20 in a rotatable manner, as illustrated in FIG. 15, instead of a through hole, the hole portion 30H may be such that a cylindrical hole having a diameter d5 is formed in the rotating body 30 and a hole having the diameter d2 smaller than the diameter d5 and leading to this cylindrical hole is formed from the surface 33 side. Here, a diameter d4 of the end portion 22 is configured to satisfy d5 > d4 > d2. Accordingly, the end portion 22 having the diameter d4 can rotate inside the rotating body 30, and the rotating body 30 is thus connected to the shaft portion 20 in a rotatable manner. To obtain the rotating body 30 in FIG. 15, for example, the rotating body 30 is configured as two portions with reference to the central axis in the width direction W (FIG. 12), then the shaft portion 20 is sandwiched between the two portions, and the two portions are joined to obtain the rotating body 30.

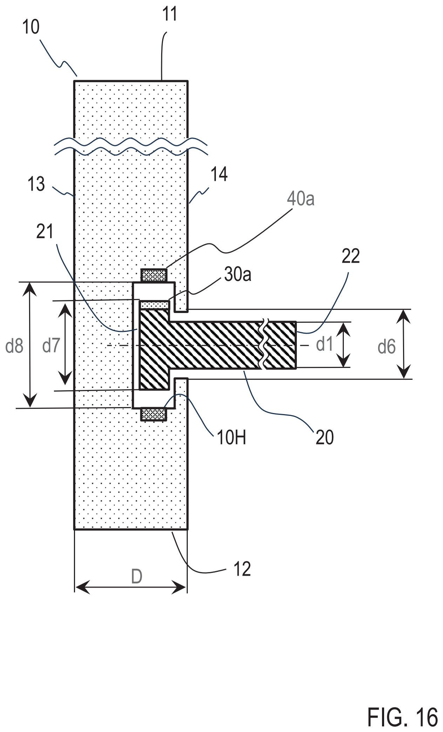

FIG. 16 is a diagram illustrating an example of a method for connecting the insertion and extraction jig 10 and the shaft portion 20 according to the modification of the first embodiment. When a magnetic sensor is used as the sensor unit 40 and the rotating body 30 and the shaft portion 20 are fixedly connected to each other, the shaft portion 20 needs to be connected to the insertion and extraction jig 10 in a rotatable manner. In this case, as illustrated in FIG. 16, the end portion 21 side of the shaft portion 20 is configured to have a diameter d7, which is larger than the shaft diameter d1 of the shaft portion 20, and a cylindrical hole having a diameter d8 larger than the diameter d7 is formed in the insertion and extraction jig 10, and a hole having a diameter d6 (d7 > d6 > d1) and leading to this cylindrical hole is formed from the surface 14 side. Accordingly, the end portion 21 of the shaft portion 20 can rotate inside the insertion and extraction jig 10, and the shaft portion 20 is connected to the insertion and extraction jig 10 in a rotatable manner. To obtain the insertion and extraction jig 10 in FIG. 16, for example, the insertion and extraction jig 10 is configured as two portions, then the shaft portion 20 is sandwiched between the two portions, and the two portions are joined to obtain the insertion and extraction jig 10, in the same manner as the above description of FIG. 15.

When the shape of FIG. 16 is adopted, the magnet 30a may be attached to the end portion 21 side instead of inside the rotating body 30, and the magnetic sensor 40a may be provided at a position where the magnetism of the magnet 30a can be detected. In the case of FIG. 16, the shaft portion 20 and the rotating body 30 are fixedly connected, making it possible to indirectly detect the movement of the rotation of the rotating body 30 based on the movement of the rotation of the magnet 30a attached to the end portion 21 side. In this configuration, it needs to be taken into account to ensure that the attachment of the magnet 30a does not cause the shaft portion 20 to resonate when the shaft portion 20 rotates.

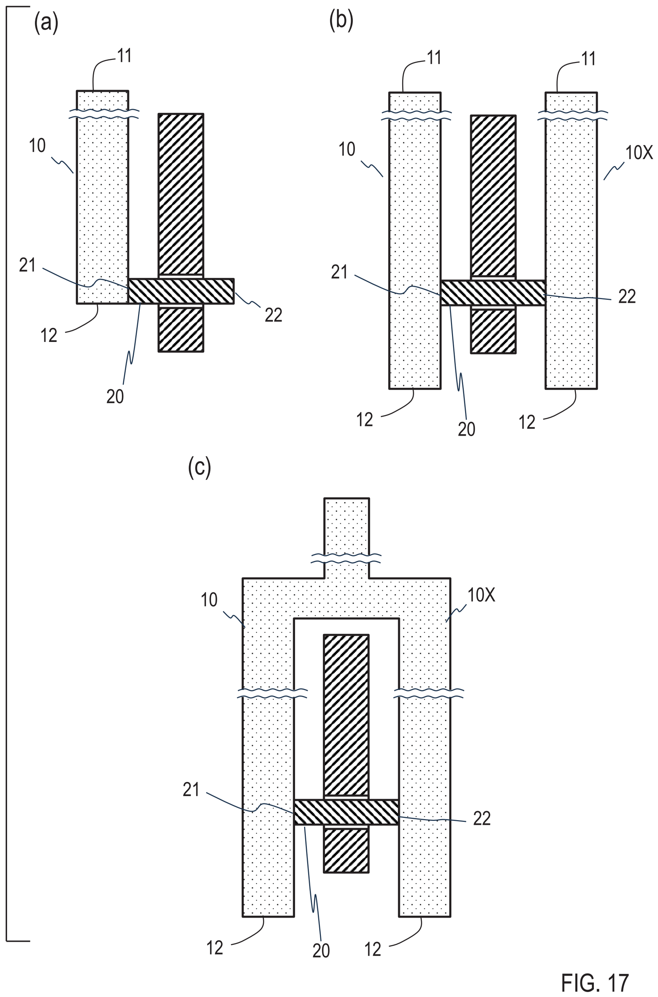

The insertion and extraction jig 10 may have a configuration as illustrated in FIG. 17. FIG. 17 is a diagram illustrating other configurations of the insertion and extraction jig 10. In FIGs. 5 and 11, the shaft portion 20 is connected to the end portion 12 side near the end portion 12 of the insertion and extraction jig 10. However, as illustrated in FIG. 17(a), the shaft portion 20 may be connected so as to protrude directly from the end portion 12 of the insertion and extraction jig 10. Alternatively, the shaft portion 20 may be connected not only to the insertion and extraction jig 10 at the end portion 21 side, but also to an insertion and extraction jig 10X, which is provided at the end portion 22 side and equivalent to the insertion and extraction jig 10, at the end portion 22 side, as illustrated in FIG. 17(b). In this case, as illustrated in FIG. 17(c), the insertion and extraction jig 10 and the insertion and extraction jig 10X may be configured such that the end portion 11 sides are joined together so that the insertion and extraction jig 10 and the insertion and extraction jig 10X are integrated.

### <Second embodiment>

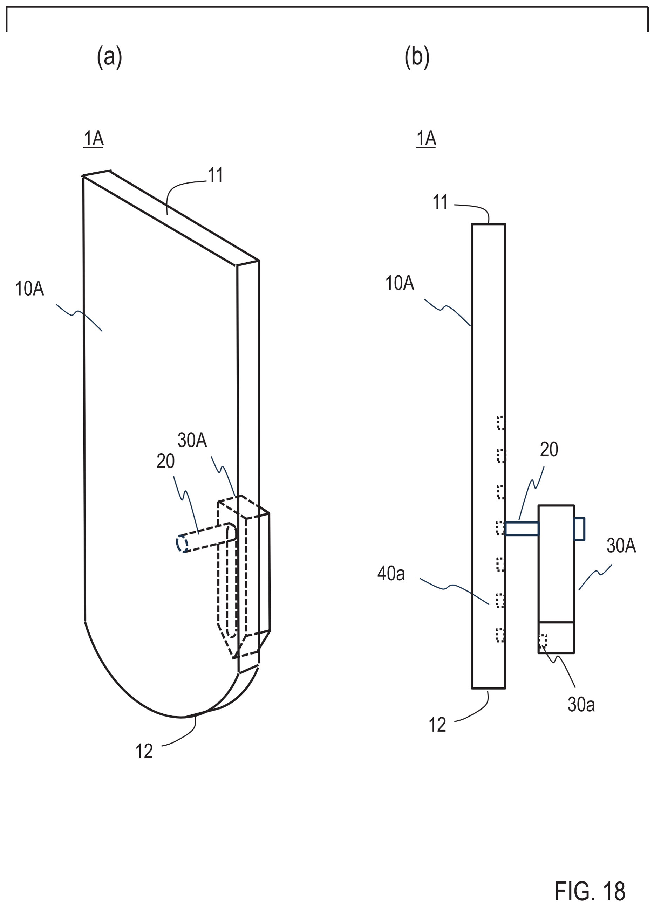

When the rotating body 30 is configured to have a specific gravity smaller than the specific gravity of the material D in the fluidization measuring instrument 1, a configuration may be adopted as that of a fluidization measuring instrument 1A illustrated in FIG. 18. For example, when the material D is fresh concrete (specific gravity S₀ = 2.3), the rotating body 30 is made of polypropylene (specific gravity S₁ = 0.9). FIG. 18(a) is a perspective view of the fluidization measuring instrument 1A according to a second embodiment and FIG. 18(b) is a side view of the same. In FIG. 18(a), the shaft portion 20 and a portion of the rotating body 30, which are not actually visible in a perspective view, are indicated by dotted lines. In FIG. 18(b), the magnetic sensors 40a and the magnet 30a, which are not actually visible in a side view, are indicated by dotted lines. The fluidization measuring instrument 1A includes an insertion and extraction jig 10A, the shaft portion 20, a rotating body 30A, and the sensor unit 40. The sensor unit 40 adopts the above-mentioned magnetic sensor system (magnetic sensor 40a).

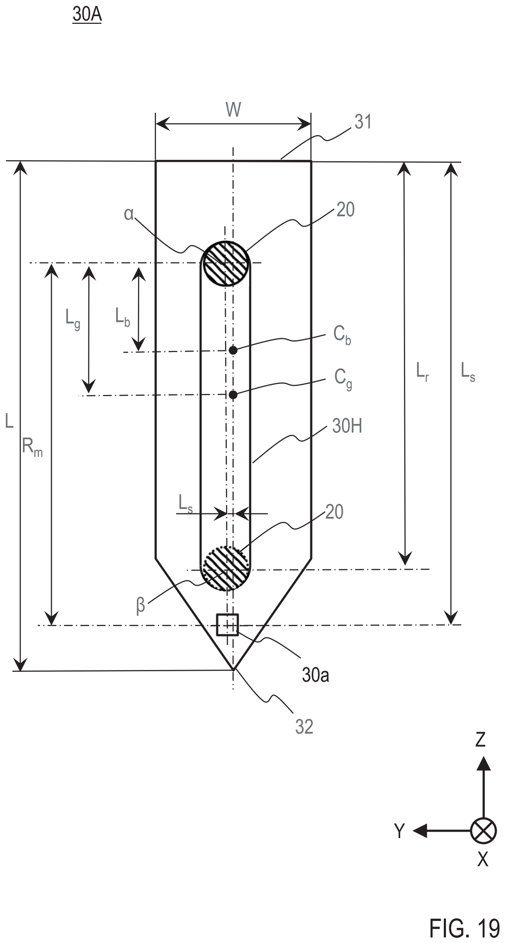

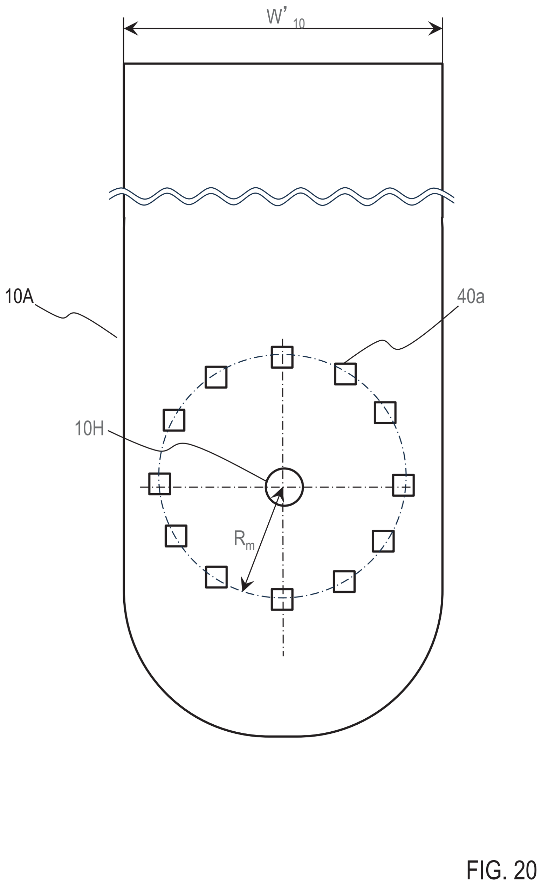

FIG. 19 is a front elevational view of the rotating body 30A viewed from an insertion and extraction jig 10A side. FIG. 20 is a front elevational view of the insertion and extraction jig 10A viewed from a rotating body 30A side.

In the fluidization measuring instrument 1, the hole portion 30H of the rotating body 30 has a circular hole shape. In the fluidization measuring instrument 1A, the shape of the hole portion 30H of the rotating body 30A is an elongated hole which allows the shaft portion 20 to move in the length direction of the rotating body 30A (Z direction and -Z direction in FIG. 19). In other words, the hole portion 30H has a shape which allows the end portion 22 side of the shaft portion 20 to move from a predetermined position (first position α) to a position (second position β) closer to the end portion 32 than the first position α. The rotating body 30A is mounted on the end portion 22 side of the shaft portion 20 through the hole portion 30H, in a manner to be rotatable about the shaft portion 20 as a rotating shaft. In the fluidization measuring instrument 1A, the width of the insertion and extraction jig 10A is widened to match a length Rₘ from the center of rotation of the rotating body 30A to the magnet 30a, which is reflected and shown as W'₁₀ in FIG. 20. Here, the rotating body 30A is connected to the shaft portion 20 in a rotatable manner and therefore, the shaft portion 20 and the insertion and extraction jig 10A are fixedly connected to each other.

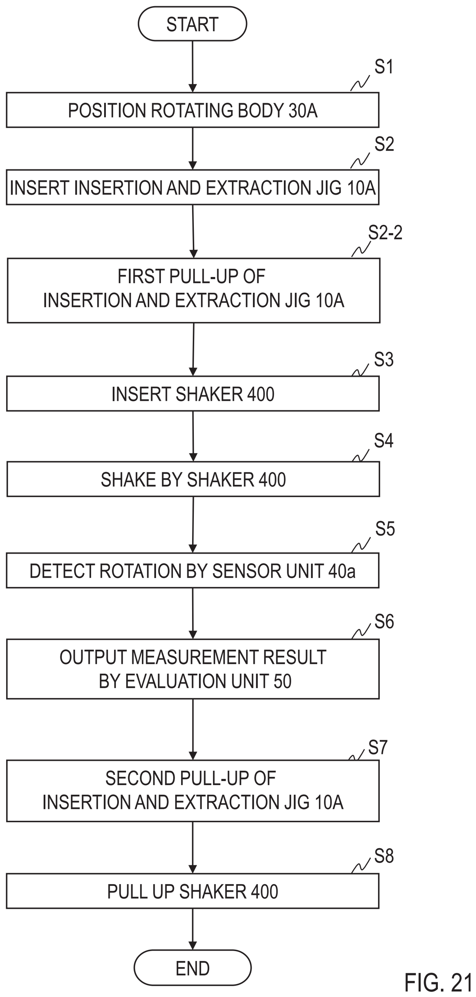

FIG. 21 is a flowchart showing a fluidization measuring method using the fluidization measuring instrument 1A. In FIG. 21, the insertion and extraction jig 10 in FIG. 4 is changed to the insertion and extraction jig 10A, the rotating body 30 is changed to the rotating body 30A, the sensor unit 40 is changed to the magnetic sensor 40a, and the notation in step S7 is changed from "pull up" to "second pull-up" as a matter of form. The fluidization measuring method of the fluidization measuring instrument 1A substantially differs from the fluidization measuring method of the fluidization measuring instrument 1 in that step S2-2 is added. The rotating body 30A has the smaller specific gravity than the material D and accordingly, when the fluidization measuring instrument 1A is inserted into the material D in step S2, the fluidization measuring instrument 1A is inserted in the depth direction in a state where the shaft portion 20 is positioned on the end portion 32 side of the rotating body 30 in the elongated hole of the hole portion 30H (second position β). That is, with the configuration of the rotating body 30A having the elongated hole, a state in which the rotating body 30A is less likely to rotate can be maintained during the insertion of the insertion and extraction jig 10A in step S2.

Once the rotating body 30A can be placed at a desired measurement position, the insertion and extraction jig 10A is pulled up by a predetermined length so that the shaft portion 20 moves to the end portion 31 side (first position α) of the hole portion 30H of the rotating body 30A (first pull-up, step S2-2). That is, the shaft portion 20 is pulled up from the second position β to the first position α. By performing step S2-2, the rotation of the rotating body 30A can be encouraged with the larger Rₘ.

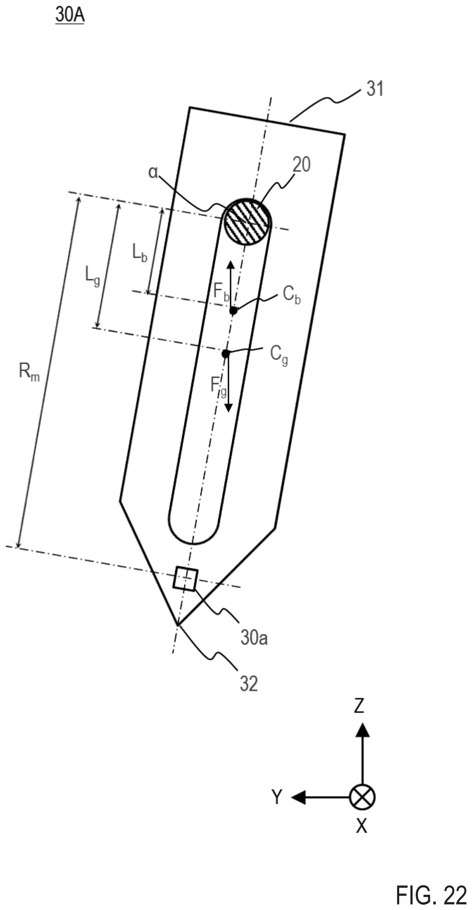

FIG. 22 is a diagram illustrating an example of a positional relationship between the center of gravity C_{g}, the center of buoyancy C_{b}, and the shaft portion 20 in the rotating body 30A. The rotating body 30A is configured so that when the rotating body 30A is entirely inserted into the material D while maintaining the first posture in which the end portion 32 is deeper than the end portion 31 in the depth direction, and when the end portion 22 side of the shaft portion 20 is positioned on the first position α, the position of the center of buoyancy C_{b} of the rotating body 30A, the position of the center of gravity C_{g} of the rotating body 30, and the first position α are set so as to change the posture from the first posture to the second posture in which the end portion 31 is deeper than the end portion 32 in the depth direction along with the rotation of the rotating body 30A associated with fluidization of the material D. The magnet 30a is embedded in the end portion 32 side of the rotating body 30A and accordingly, the large distance Rₘ can be secured between the shaft portion 20 and the magnet 30a, which facilitates circumferential rotational movement with the wider radius Rₘ than that of the fluidization measuring instrument 1. When the longer radius Rₘ than that of the rotating body 30 in the first embodiment can be secured, the fluidization measuring instrument 1A can detect the rotational movement of the rotating body 30A more accurately than the rotational movement of the rotating body 30 of the fluidization measuring instrument 1.

The embodiments and the modification of the present disclosure have been described above. The material D in the present embodiments is described employing fresh concrete as an example, but the material D is not limited to fresh concrete as long as it is fluidized by shaking or heating. For example, it can be foodstuffs such as curry roux or other materials that are fluidized by heating.

Further, instead of adopting the magnetic sensor system for the sensor unit 40, a combination of a light receiving element and a light emitting element is applicable. For example, a light emitting element may be used instead of the magnet, and a light receiving element may be used instead of the magnetic sensor. When an accelerometer is adopted as the sensor unit 40, the accelerometer may be mounted on the rotating body 30 and a result may be sent to the evaluation unit 50. The insertion and extraction jig 10 or the insertion and extraction jig 10A may have the function of the evaluation unit 50. In addition, it goes without saying that the shape, the size, the material, and so forth of the insertion and extraction jig 10, the shaft portion 20, the rotating body 30, and the sensor unit 40 described in the present embodiments can be appropriately modified without departing from the spirit of the present disclosure.

### [DESCRIPTION OF REFERENCE NUMERALS]

- 1, 1A: fluidization measuring instrument
- 10, 10A: insertion and extraction jig
- 10H, 30H: hole portion
- 11, 12, 21, 22, 31, 32: end portion
- 13, 14, 33, 34: surface
- 20: shaft portion
- 200: formwork
- 30, 30A: rotating body
- 30a: magnet
- 310: first portion
- 320: second portion
- 40: sensor unit
- 40a: magnetic sensor
- 400: shaker
- 50: evaluation unit
- 90A: upper layer
- 90B: lower layer
- C_{g}: center of gravity
- C_{b}: center of buoyancy
- d1: shaft diameter
- d2: to d8 diameter
- D: material
- F_{b}: buoyancy
- F_{g}: gravity
- L, LL, L_{b}, L_{g}, Lᵣ, Lₛ, Rₘ, W, W₁₀: length
- S₀, S₁: specific gravity
- T: mounting position
- α: first position
- β: second position

## Claims

1. A fluidization measuring instrument for measuring a degree of fluidization of a material which is fluidized by shaking or heating, the fluidization measuring instrument comprising:
an insertion and extraction jig which includes a first end portion and a second end portion, the second end portion being positioned opposite to the first end portion and being to be inserted into the material before performing a process of shaking or heating;
a shaft portion which includes one end and another end, the another end being positioned opposite to the one end, and which is inserted into the material together with the insertion and extraction jig with the one end connected to the insertion and extraction jig;
a rotating body which includes a third end portion and a fourth end portion, the fourth end portion being positioned opposite to the third end portion, and which is connected with the another end of the shaft portion, at a mounting position provided between the third end portion and the fourth end portion, so as to be rotatable with respect to the insertion and extraction jig using the shaft portion as a rotating shaft; and
a sensor unit which is capable of detecting rotation of the rotating body, wherein
the rotating body is configured so that when the rotating body is entirely inserted into the material while maintaining a first posture, in which the fourth end portion is deeper than the third end portion in a depth direction, a position of a center of buoyancy of the rotating body, a position of a center of gravity of the rotating body, and the mounting position are set so as to change a posture from the first posture to a second posture, in which the third end portion is deeper than the fourth end portion in the depth direction, along with the rotation associated with fluidization of the material.

2. The fluidization measuring instrument according to Claim 1, wherein in the rotating body in the first posture, the center of buoyancy is positioned closer to the fourth end portion than the mounting position and the center of gravity is positioned closer to the third end portion than the mounting position.

3. The fluidization measuring instrument according to Claim 1, wherein in the rotating body in the first posture, the mounting position is positioned closer to the fourth end portion than the center of buoyancy and the center of gravity, and a ratio between a length from the center of buoyancy to the mounting position and a length from the center of gravity to the mounting position satisfies a predetermined first relationship.

4. The fluidization measuring instrument according to Claim 1, wherein in the rotating body in the first posture, the mounting position is positioned closer to the third end portion than the center of buoyancy and the center of gravity, and a ratio between a length from the center of buoyancy to the mounting position and a length from the center of gravity to the mounting position satisfies a predetermined second relationship.

5. The fluidization measuring instrument according to Claim 2, wherein a center of the mounting position is separated from a straight line passing through the center of buoyancy and the center of gravity.

6. The fluidization measuring instrument according to Claim 2, wherein the one end of the shaft portion is fixedly connected with the insertion and extraction jig.

7. The fluidization measuring instrument according to Claim 2, wherein the rotating body is fixedly connected with the another end of the shaft portion.

8. The fluidization measuring instrument according to Claim 7, wherein the shaft portion is connected to the insertion and extraction jig in a manner to be rotatable about a shaft direction.

9. The fluidization measuring instrument according to Claim 8, wherein the sensor unit is mounted on the rotating body.

10. A fluidization measuring instrument for measuring a degree of fluidization of a material which is fluidized by shaking or heating, the fluidization measuring instrument comprising:
an insertion and extraction jig which includes a first end portion and a second end portion, the second end portion being positioned opposite to the first end portion and being to be inserted into the material before performing a process of shaking or heating;
a shaft portion which includes one end fixedly connected to the insertion and extraction jig and another end positioned opposite to the one end, and which is inserted into the material together with the insertion and extraction jig;
a rotating body which includes a third end portion, a fourth end portion positioned opposite to the third end portion, and an elongated hole portion allowing another end side of the shaft portion to move from a first position to a second position, the second position being closer to the fourth portion than the first position, is mounted on the another end side of the shaft portion through the elongated hole portion in a manner to be rotatable about the shaft portion as a rotating shaft, and is configured to have a specific gravity smaller than a specific gravity of the material; and
a sensor unit which is capable of detecting rotation of the rotating body, wherein
the rotating body is configured so that when the rotating body is entirely inserted into the material while maintaining a first posture, in which the fourth end portion is deeper than the third end portion in a depth direction, and when the another end side of the shaft portion is positioned on the first position, a position of a center of buoyancy of the rotating body, a position of a center of gravity of the rotating body, and the first position are set so as to change a posture from the first posture to a second posture, in which the third end portion is deeper than the fourth end portion in the depth direction, along with the rotation associated with fluidization of the material.
